# EUROPÄISCHE PATENTSCHRIFT

(11) **EP 0 623 600 B1**
(45) Veröffentlichungstag und Bekanntmachung des Hinweises auf die Patenterteilung: **13.05.1998**
(21) Anmeldenummer: 94103293.0
(22) Anmeldetag: 04.03.1994
(51) Int. Cl.: C07D 231/38

(54) **Verfahren zur Herstellung von 3-Amino-5-methylpyrazol**
Process for the preparation of 3-amino-5-methylpyrazole
Procédé pour la préparation de 3-amino-5-méthylpyrazol

(30) Priorität: 05.05.1993 DE 4314851
(43) Veröffentlichungstag der Anmeldung: 09.11.1994
(73) Patentinhaber: HÜLS AKTIENGESELLSCHAFT, 45764 Marl (DE)
(72) Erfinder: Muhr, Jürgen, Dr., D-53347 Alfter (DE); Feld, Marcel, Dr., D-51147 Köln (DE)

(56) Entgegenhaltungen:
- EP-A- 0 075 773
- EP-A- 0 334 133
- GB-A- 1 268 608
- CHEMICAL ABSTRACTS, vol. 118, no. 25, 21. Juni 1993, Columbus, Ohio, US; abstract no. 254928u, Seite 887 ;Spalte 1 ;
- CHEMICAL ABSTRACTS, vol. 118, no. 9, 1. März 1993, Columbus, Ohio, US; abstract no. 80932y, Seite 838 ;Spalte 1 ;
- JOURNAL OF HETEROCYCLIC CHEMISTRY Bd. 11, Nr. 3 , Juni 1974 , PROVO US Seiten 423 - 429 E. ALCADE ET AL. 'Etude de la réaction du B-aminocrotonitrile et du a-formyl phénylacétonitrile avec l'hydrazine: synthèse d'amino-7 pyrazolo[1,5-a]pyrimidines'
- BERICHTE DER DEUTSCHEN CHEMISCHEN GESELLSCHAFT Bd. 42 , 1909 , WEINHEIM DE Seiten 59 - 68 L. CLAISEN 'Über a-Methyl-isoxazol'

## Beschreibung

Die Erfindung betrifft die Herstellung von 3-Amino-5-methylpyrazol (3.5-AMP) oder dessen Salzen durch Umsetzung von Alkali-Cyanaceton mit Hydraziniumsalzen.

3.5-AMP findet als Vorstufe für "Magenta-Coupler", d. h. Fuchsin, in photographischen Materialien, sowie bei der Synthese von Pharmaka Verwendung.

Bekannte Synthesen von 3.5-AMP erfolgen aus Hydrazinhydrat und 3-Aminocrotononitril (JP 63239272; Derwent OD-326015/88) oder 2-Iminobutyronitril (SU 1413106; Derwent OD-038663/89).

Nach JP 92/253 964 und JP 92/275 277 ist auch die Synthese von Aminopyrazolen aus einem Isooxazol und Hydrazinhydrat bzw. Hydrazin in Gegenwart von Alkaliverbidungen bekannt.

Diese Verfahren gehen von schwer zugänglichen bzw. chemisch nicht stabilen Ausgangsstoffen aus und/oder sind vielstufige, aufwendige Synthesen mit unzureichenden Ausbeuten.

Nach EP-A-0 334 133 kann 3-Amino-5-methylpyrazol aus Cyanaceton und Hydrazinhydrat hergestellt werden, wobei das angegebene Verfahren das Produkt nur in unbefriedigenden Ausbeuten liefert.

Darüber hinaus ist nach Chem. Ber. 42, 59-68 (1909) bekannt, daß Kaliumcyanaceton mit Phenylhydrazin zu einem Phenylhydrazon reagieren kann. Nach EP-A-0 075 773 kann auch 3-Aminophenyl-trimethyl-ammoniumhydrogensulfat zunächst diazotiert und dann bei pH 1 mit dem Natriumsalz von Cyanaceton zu einem Pyrazolderivat umgesetzt werden. Wegen der erheblichen Struktur- und Stabilitätsunterschiede zum Hydrazin liefern diese Schriften kaum Hinweise für die Herstellung von 3-Amino-5-methylpyrazol.

In J. Heterocyclic Chem. 11, 423-429 (1974) wird außerdem beschrieben, daß man α-Formylphenylacetonitril mit Hydrazin in ein Pyrazolderivat überführen kann. Hier ist der Reaktionspartner des Hydrazins durch die Aldehydgruppe sehr reaktiv. Außerdem begünstigt die Phenylgruppe eine Ringschlußreaktion. Eine Aussage für das Verhalten von Cyanaceton kann dieser Schrift dabei nicht entnommen werden.

Es bestand somit die Aufgabe, diese Nachteile zu vermeiden und 3.5-AMP aus einfach zugänglichen Ausgangsstoffen mit hoher Ausbeute und Reinheit auf einem einfach ausführbaren Weg herzustellen.

Diese Aufgabe wird durch das vorliegende Verfahren gelöst, wobei ein Alkalicyanaceton mit einem Hydraziniumsalz einer Mineralsäure durch Abspaltung von Wasser und Bildung von 3.5-AMP und einem Alkalisalz zur Reaktion gebracht wird.

Die Alkalisalze von Cyanaceton H₃C-CO-CH₂-CN (Acetessigsäurenitril) sind nach Claisen durch Spaltung von 5-Methylisooxazol zugänglich (Besten H₃, 659).

Die Kondensation aus Alkalicyanaceton, besonders Natriumcyanaceton (NaCyA) oder dem K-Salz, mit Hydraziniumsalzen, wie Hydraziniumhydrochlorid, wird bevorzugt in einem homogenen oder zweiphasigen Lösungsmittelgemisch ausgeführt. Geeignete Lösungsmittel sind überraschend besonders Wasser, auch organische Lösungsmittel, wie Alkohol, Ether oder Kohlenwasserstoffe mit 2 bis 8 C-Atomen sowie Alkylaromaten, besonders Toluol oder Xylol, gegebenenfalls zusammen mit Wasser.

Bevorzugt wird bei Reaktionstemperatur die Lösung oder Suspension eines der Reaktanden zu einer Lösung oder Suspension des anderen Reaktanden zugesetzt. Die Reaktionspartner können auch gelöst oder suspendiert gemeinsam vorgelegt werden. Wasser kann während der Reaktion oder danach ganz oder teilweise abdestilliert bzw. mittels eines Schleppmittels azeotrop ausgekreist werden. Die Dosierung der Hydrazinkomponente zum erwärmten Reaktionsgemisch ist aus Sicherheitsgründen von Vorteil. Eine kontinuierliche Verfahrensweise ist möglich.

Die Reaktion erfolgt bei Temperaturen von 10 bis 200 °C, bevorzugt bei der Siedetemperatur des verwendeten Solvens unter Normaldruck oder gegebenenfalls unter einem Druck bis 10 bar. In wäßrigem Medium sind 30 bis 100 °C bevorzugt.

Bei Durchführung mit wäßrigem Hydrazinium-monohydrochlorid z. B. als Lösung wird vorteilhaft der pH-Wert auf 1 bis 2 mit Säuren, z. B. Salzsäure, eingestellt. Dafür sind Mengen bis 1 mol-% ausreichend.

Als Schleppmittel einer azeotropen Destillation eignen sich Alkylbenzole, wie Xylol oder Toluol, die bereits während der Kondensation anwesend sein können. Der Zusatz wasserbindender Hilfsstoffe vor, während oder nach der Reaktion ist möglich, auch die Umsetzung in wasserfreiem Medium.

Außer 3.5-AMP entsteht ein Alkalisalz, zumeist NaCl. Dieses Alkalisalz kann bei Umsetzung in hydrophobem Solvens, wie Toluol, auch nach Auskreisen des Wassers, eine nicht filtrierbare Suspension in dem niedrig schmelzenden 3.5-AMP bilden.

Die Isolierung des Produkts kann aber einfach dadurch realisiert werden, daß nach Abtrennung des Wassers das Alkalisalz durch Zugabe niedermolekularer Alkohole oder Ketone, vorzugsweise Methanol und insbesondere Ethanol, in Gegenwart oder nach Abdestillieren eines gegebenenfalls verwendeten Schleppmittels in gut filtrierbarer Form zur Kristallisation gebracht und durch eine übliche Fest/Flüssig-Trennung abgetrennt wird. Die erfindungsgemäße Kondensationsreaktion, wie auch das Herausdestillieren des (Reaktions)wassers, kann auch direkt mit den zur Fällung des Alkalisalzes verwendeten Solventien, vorzugsweise mit verzweigten oder geradkettigen Alkoholen mit einem bis 5 Kohlenstoffatomen, besonders bevorzugt in/mit Ethanol, durchgeführt werden. Nach Abtrennung des Alkalisalzes kann das Zielprodukt aus dem Filtrat vorzugsweise durch Destillation gewonnen werden. Es besteht allerdings auch die Möglichkeit, das 3.5-AMP durch Zufügen einer Mineral- oder Carbonsäure zum Filtrat in das entsprechende Salz zu überführen und in dieser Form zu isolieren.

### Beispiel 1

1 mol Hydrazinium-monohydrochlorid als 40 Gew.-%ige wäßrige Lösung und 200 ml Toluol werden zum Rückflußsieden erhitzt und innerhalb von 2½ Stunden mit 1 mol NaCyA als 30 Gew.-%ige wäßrige Lösung versetzt. Nachdem restliches Wasser ausgekreist worden ist, destilliert man Toluol ab und fällt NaCl durch Zugabe von Ethanol. Nach Abfiltern engt man ein und reinigt via Vakuumdestillation:
Kp (2 mm) 128 °C.
Ausbeute 72 g (74 % d. Th.), Reinheit über 99 %.

### Beispiel 2

1,43 mol Hydrazinium-monohydrochlorid als 40 Gew.-%ige wäßrige Lösung werden mit katalytischen Mengen konzentrierter HCl auf pH 1 bis 2 eingestellt. Sodann dosiert man äquimolare Mengen NaCyA bei 30 °C innerhalb von 45 Minuten zu. Nach 4½ Stunden Nachreaktion versetzt man die Reaktionslösung mit 650 ml Toluol und entfernt das Wasser durch Azeotrop-Destillation. Durch Zugabe des gleichen Volumens an Ethanol fällt man NaCl, filtriert ab, engt ein und destilliert im Vakuum.
Ausbeute 100,6 g (71 % d. Th.), Reinheit über 98 %.

### Beispiel 3

1 mol einer 40 Gew.-%igen wäßrigen Hydrazinium-monohydrochlorid-Lösung werden bei 35 °C innerhalb von 2 Stunden mit 1 mol NaCyA versetzt. Nach 4½ Stunden Nachreaktion fügt man 400 ml Toluol hinzu, kreist zunächst das Wasser aus und destilliert Toluol ab. Aus dem zähflüssigen Rückstand fällt man NaCl mit 200 ml Ethanol, filtriert ab und entfernt Ethanol im Vakuum.
Ausbeute 99 g (88,6 % d. Th.), Reinheit über 89%.

### Beispiel 4

Eine Suspension von 1 mol NaCyA und 1 mol Hydrazinium-monohydrochlorid in 300 ml Toluol wird am Wasserabscheider bis zur Konstanz der abgeschiedenen Wassermenge auf Rückflußtemperatur erhitzt. Nach Abkühlen der Reaktionsmasse fällt man NaCl mit Ethanol, filtriert ab und arbeitet destillativ auf.
Ausbeute 71 g (72 % d. Th.), Reinheit über 98 %.

### Beispiel 5

Eine Suspension aus 105,1 g (1 Mol) NaCyA in Toluol wird auf Siedetemperatur erhitzt und innerhalb von 2 Stunden mit 1 mol 40 Gew.-%iger Hydrazinium-monohydrochlorid-Lösung versetzt, wobei kontinuierlich Wasser ausgekreist wird. Nach vollständiger Abtrennung des Wassers wird wie in Beispiel 1 aufgearbeitet.
Ausbeute 85,2 g (83,3 % d. Th.), Reinheit über 95 %.

## Patentansprüche

1. Verfahren zur Herstellung von 3-Amino-5-methylpyrazol,
dadurch gekennzeichnet,
daß ein Alkali-Cyanaceton mit einem Hydraziniumsalz einer Mineralsäure umgesetzt wird.

2. Verfahren nach Anspruch 1,
dadurch gekennzeichnet,
daß die Umsetzung in einem Lösungsmittel oder Suspensionsmittel erfolgt.

3. Verfahren nach Anspruch 2,
dadurch gekennzeichnet,
daß als Lösungsmittel oder Suspensionsmittel Wasser, organische Lösungsmittel oder deren Mischungen verwendet werden.

4. Verfahren nach einem der Ansprüche 1 bis 3,
dadurch gekennzeichnet,
daß gebildetes oder zugesetztes Wasser während und/oder nach der Reaktion entfernt wird.

5. Verfahren nach mindestens einem der Ansprüche 1 bis 3,
dadurch gekennzeichnet,
daß die Reaktion im Temperaturbereich von 10 bis 200 °C, vorzugsweise von 20 bis 60 °C, bei Normaldruck durchgeführt wird.

6. Verfahren nach einem der Ansprüche 1 bis 5,
dadurch gekennzeichnet,
daß die bei der Umsetzung gebildeten Alkalisalze des mit dem Hydrazin-Salz eingebrachten Säurerests nach Abtrennung des bei der Reaktion gebildeten und gegebenenfalls zusätzlich eingebrachten Wassers, durch Zusatz eines niedermolekularen Alkohols oder Ketons zur Kristallisation gebracht und durch Filtration abgetrennt werden.

## Claims

1. A process for preparing 3-amino-5-methylpyrazole, characterized in that an alkali metal cyanoacetone is reacted with a hydrazinium salt of a mineral acid.

2. A process according to claim 1 characterized in that the reaction proceeds in a solvent or a suspension medium.

3. A process according to claim 2, characterized in that water, organic solvents or mixtures thereof are used as solvent or suspension medium.

4. A process according to any one of claims 1 to 3, characterized in that water formed or added is removed during and/or after the reaction.

5. A process according to at least one of claims 1 to 3, characterized in that the reaction is carried out in the temperature range of from 10 to 200°C, preferably from 20 to 60°C, at atmospheric pressure.

6. A process according to any one of claims 1 to 5, characterized in that the alkali metal salts of the acid radical introduced by the hydrazinium salt which are formed during the reaction are crystallized, after separating off the water formed or additionally introduced, by addition of a low molecular weight alcohol or ketone and are separated off by filtration.

## Revendications

1. Procédé de fabrication de 3-amino-5-méthyl-pyrazole,
caractérisé en ce qu'
on fait réagir une cyanacétone alcaline avec un sel d'hydrazinium d'un acide minéral.

2. Procédé selon la revendication 1,
caractérisé en ce qu'
on procède à la réaction dans un solvant ou agent de suspension.

3. Procédé selon la revendication 2,
caractérisé en ce qu'
on utilise comme solvant ou agent de suspension l'eau, les solvants organiques ou leurs mélanges.

4. Procédé selon l'une des revendications 1 à 3,
caractérisé en ce qu'
on enlève l'eau formée ou ajoutée pendant et/ou après la réaction.

5. Procédé selon au moins l'une des revendications 1 à 3,
caractérisé en ce qu'
on conduit la réaction dans un intervalle de température de 10 à 200°C, de préférence de 20 à 60°C, à pression normale.

6. Procédé selon l'une des revendications 1 à 5,
caractérisé en ce qu'
on fait se cristalliser les sels alcalins, formés lors de la réaction, du radical acide introduit avec le sel d'hydrazine, après séparation de l'eau formée lors de la réaction et éventuellement ajoutée en outre, par addition d'un alcool ou d'une cétone à bas point moléculaire, et en ce qu'on sépare par filtration.
